(19) Europäisches Patentamt
European Patent Office
Office européen des brevets

(11) Numéro de publication: **0 015 845**
**B1**

(12) # FASCICULE DE BREVET EUROPEEN

(45) Date de publication du fascicule de brevet: **09.12.81**

(51) Int. Cl.³: **C 07 C 69/587,** C 07 C 67/347

(21) Numéro de dépôt: **80400296.2**

(22) Date de dépôt: **04.03.80**

(54) **Nouveaux esters insaturés et leur procédé de préparation.**

(30) Priorité: **12.03.79 FR 7906214**

(43) Date de publication de la demande: **17.09.80 Bulletin 80/19**

(45) Mention de la délivrance du brevet: **09.12.81 Bulletin 81/49**

(84) Etats contractants désignés: **AT BE CH DE FR GB IT LU NL SE**

(56) Documents cités: **US-A-4 130 659**

(73) Titulaire: **RHONE-POULENC INDUSTRIES, 22, avenue Montaigne, F-75008 Paris (FR)**

(72) Inventeur: **Morel, Didier, 19, avenue Jean Mermoz, F-69008 Lyon (FR)**

(74) Mandataire: **Cazes, Jean-Marie et al, RHONE POULENC Service Brevets Chimie et Polymères B.P. 753, F-75360 Paris Cedex 08 (FR)**

EP 0 015 845 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG.

Nouveaux esters insaturés et leur procédé de préparation

La présente invention a pour objet de nouveaux esters insaturés; elle concerne pus particulièrement de nouveaux esters A COO $R_1$ dans lesquels le groupement A est un groupement hydrocarboné contenant trois doubles liaisons. L'invention concerne également un procédé de préparation de ces esters insaturés.

On connait dans l'art antérieur des esters insaturés de formule R' COO B dans lesquels c'est le groupement B qui contient trois doubles liaisons (brevet français 71 027 752 publié sous le numéro 2 077 072).

Ces esters peuvent uniquement être untilisés comme intermédiaires pour la préparation d'alcools.

La demanderesse a maintenant découvert de nouveaux esters qui peuvent être utilisés comme intermédiaires pour la préparation d'alcools après hydrogénation et hydrogenolyse et de savons après hydrogénation et saponification.

La présente invention a donc pour objet de nouveaux esters insaturés de formule A COO $R_1$ caractérisés en ce que $R_1$ représente un groupement alkyle ayant 1 à 6 atomes de carbone et en ce que A est choisi parmi le groupe comprenant les restes hydrocarbonés de formule:

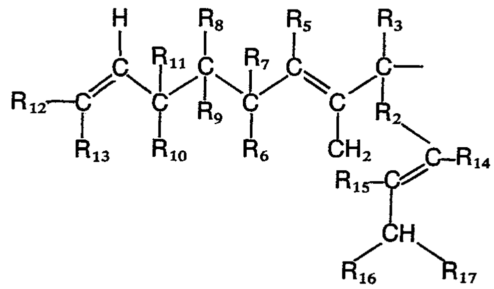

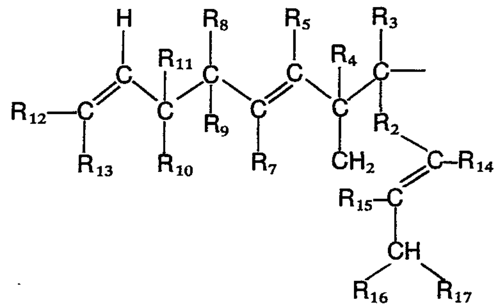

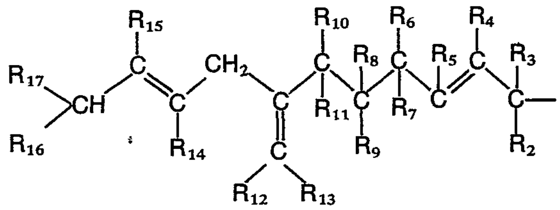

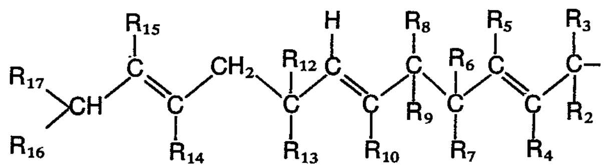

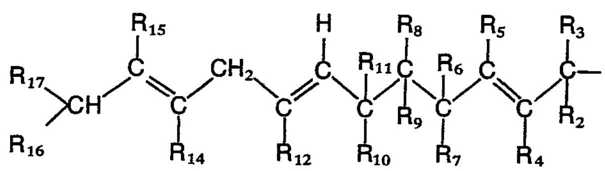

dans lesquels $R_2$ à $R_{17}$ identiques ou différents représentent un atome d'hydrogène ou un radical alkyle ayant de 1 à 3 atomes de carbone.

Un objet particulier de la présente invention est un ester de formule:

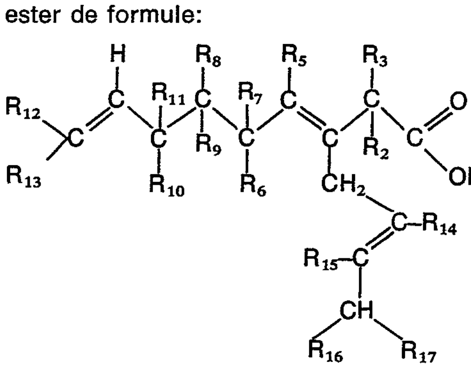

(Ia)

ou

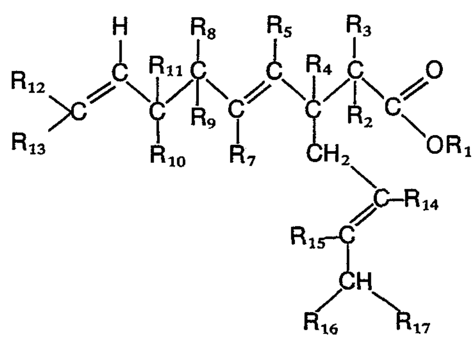

(Ib)

dans laquelle $R_1$ représente un radical alkyle ayant de 1 à 6 atomes de carbone et $R_2$ à $R_{17}$ identiques ou différents sont un atome d'hydrogène ou un radical alkyle ayant de 1 à 3 atomes de carbone.

L'invention concerne plus particulièrement parmi les esters Ia et Ib, les esters pour lesquels $R_1$ représente un radical alkyle ayant de 1 à 4 atomes de carbone et $R_2$ à $R_{17}$ représentent chacun un atome d'hydrogène.

Un autre objet particulier de l'invention est un ester de formule:

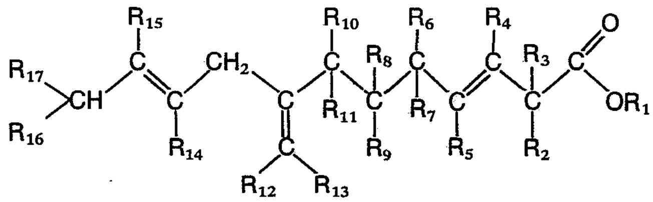

(II)

dans laquelle $R_1$ est un radical alkyle ayant de 1 à 6 atomes de carbone et $R_2$ à $R_{17}$ identiques ou différents sont un atome d'hydrogène ou un radical alkyle ayant de 1 à 3 atomes de carbone.

Parmi les esters de formule (II), l'invention vise plus particulièrement ceux pour lesquels $R_1$ représente un radical alkyle ayant de 1 à 4 atomes de carbone et $R_2$ à $R_{17}$ représentent chacun un atome d'hydrogène.

Un autre objet particulier de l'invention est un ester de formule:

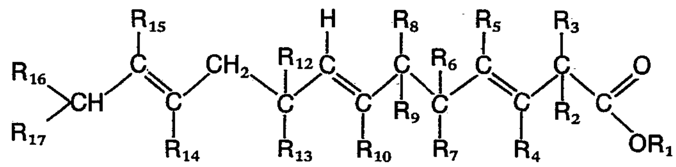

(IIIa)

ou

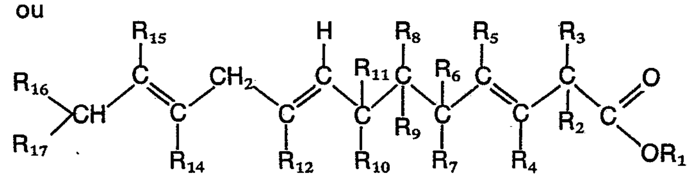

(IIIb)

dans lesquelles $R_1$ représente un radical alkyle ayant de 1 à 6 atomes de carbone et $R_2$ à $R_{17}$ identiques ou différents sont un atome d'hydrogène ou un radical alkyle ayant de 1 à 3 atomes de carbone.

Parmi les esters de formule IIIa et IIIb, l'invention vise plus particulièrement ceux pour lesquels $R_1$ représente un radical alkyle ayant de 1 à 4 atomes de carbone et $R_2$ à $R_{17}$ représentent chacun un atome d'hydrogène.

L'invention concerne également un procédé de préparation des composés de formule Ia, Ib, II, IIIa et IIIb caractérisé en ce qu'on fait réagir de formule:

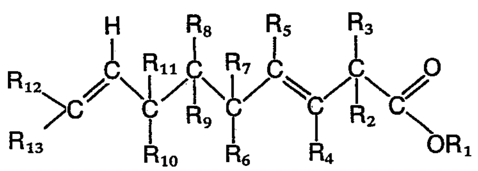

(IV)

dans laquelle $R_1$ représente un radical alkyle ayant de 1 à 6 atomes de carbone

$R_2$ $R_3$ $R_5$ $R_8$ et $R_9$ identiques ou différents sont un atome d'hydrogène ou un radical alkyle ayant de 1 à 3 atomes de carbone

$R_4$ $R_6$ et $R_7$ représentent un atome d'hydrogène ou un radical alkyle ayant de 1 à 3 atomes de carbone, au moins l'un d'entre eux étant un atome d'hydrogène

$R_{10}$ $R_{11}$ $R_{12}$ et $R_{13}$ représentent un atome d'hydrogène ou un radical alkyle ayant de 1 à 3 atomes de carbone, au moins l'un d'entre eux étant un atome d'hydrogène avec un butadiène-1,3 de formule:

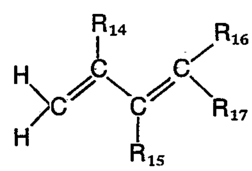

(V)

dans laquelle

$R_{14}$ $R_{15}$ $R_{16}$ ou $R_{17}$ identiques ou différents représentent chacun un atome d'hydrogène ou un radical alkyle, en présence d'un composé du rhodium et d'un alcool.

Le procédé selon l'invention est de préférence mis en œuvre en utilisant un composé de formule IV dans lequel $R_1$ représente un radical alkyle ayant de 1 à 4 atomes de carbone et $R_2$ à $E_{17}$ sont chacun un atome d'hydrogène.

Parmi ceux-ci, on peut citer:

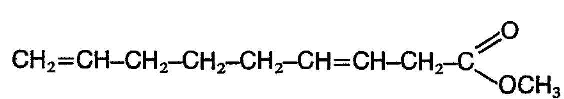

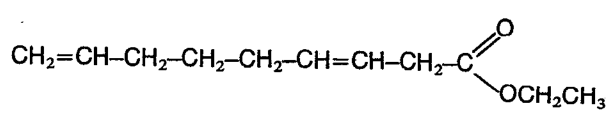

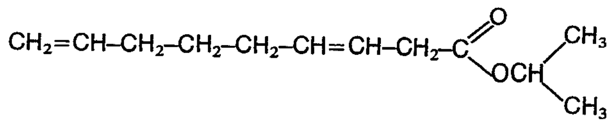

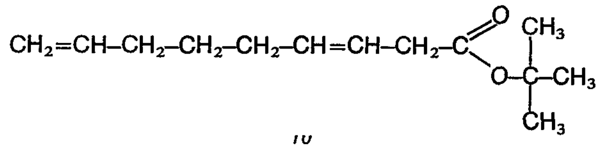

avec un composé de formule V dans lequel $R_{14}$ à $R_{17}$ sont un atome d'hydrogène, c'est-à-dire avec le butadiène-1,3.

La demanderesse a constaté d'une façon surprenante que lorsqu'on met en œuvre simultanément au composé du rhodium et à l'alcool, un composé trivalent du phosphore, on favorise la formation de l'ester de formule II. On utilise de préférence le composé du rhodium et le composé trivalent du phosphore en quantités telles que le rapport atomique

$$\frac{\text{phosphore trivalent}}{\text{rhodium}} \geqq, 1,5.$$

Encore plus préférentiellement, ce rapport est égal à environ 3. La demanderesse a également constaté que la présence du composé trivalent du phosphore diminue la quantité de produits lourds formés (obligomères du butadiène et condensats du composé IV notamment).

Lorsqu'on ne rajoute pas, lors de la mise en œuvre de la réaction le composé trivalent du phosphore, on favorise la formation des composés I et III.

On utilise de préférence comme alcool, un alcool de formule R–OH dans laquelle R est un radical alkyle ayant de 1 à 6 atomes de carbone. Encore plus préférentiellement, on utilise l'alcool éthylique.

Le composé du rhodium est choisi préférentiellement parmi les sels d'acides inorganiques du rhodium. Parmi ceux-ci le chlorure de rhodium Rh $Cl_3$ est particulièrement préféré.

Le composé trivalent du phosphore est choisi préférentiellement parmi les aryl et alkyl phosphines, comme par exemple la triphénylphosphine et la bis(diphénylphosphino)-1,2 éthylène de formule:

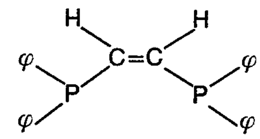

Le rapport molaire du composé de formule IV au composé du rhodium est de préférence compris entre environ 200 et environ 2000 et le rapport molaire de l'alcool au composé du rhodium est de préférence compris entre environ 10 et environ 100.

Parmi les composés selon l'invention, on peut citer:

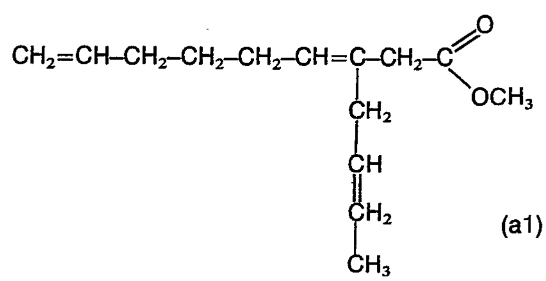

(a1)

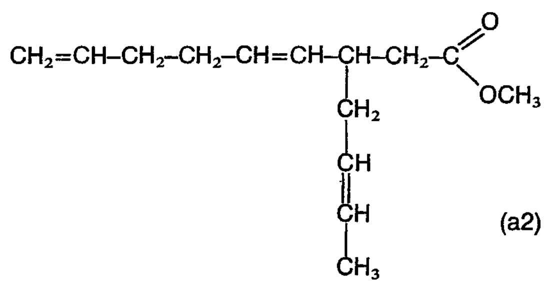

(a2)

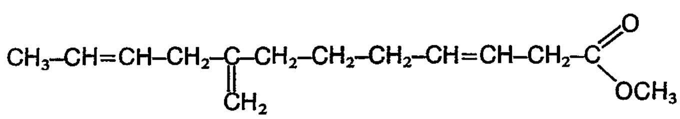

(a3)

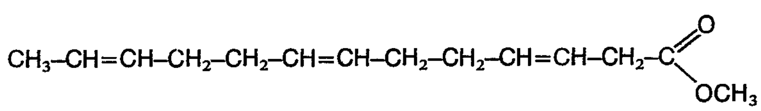

(a4)

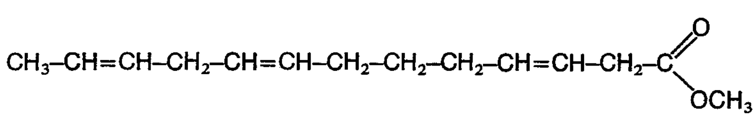

a5)

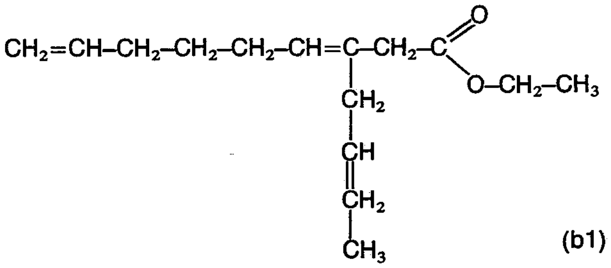

(b1)

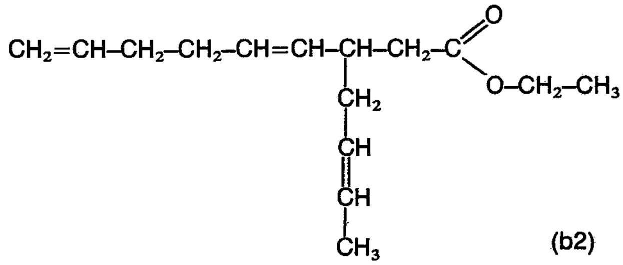

(b2)

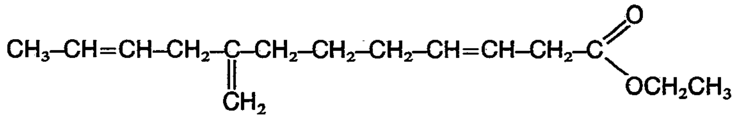

(b3)

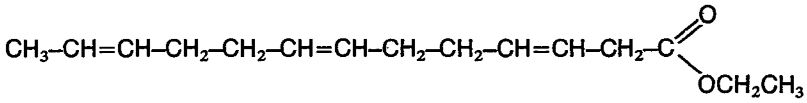

(b4)

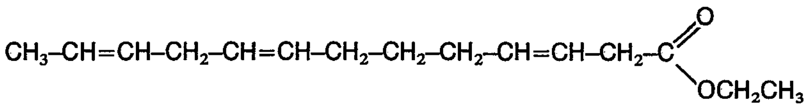

(b5)

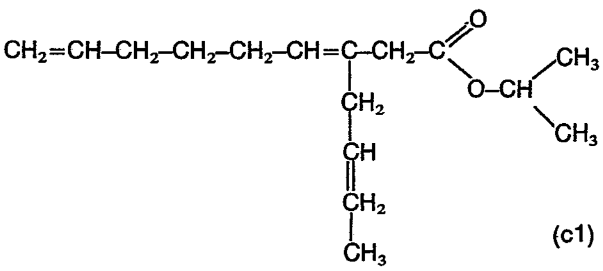

(c1)

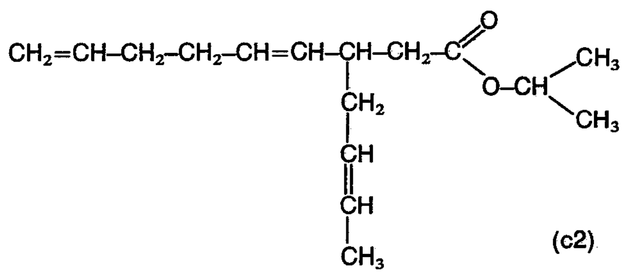

(c2)

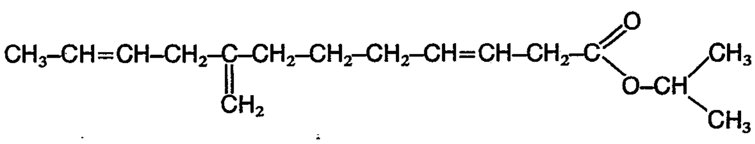

(c3)

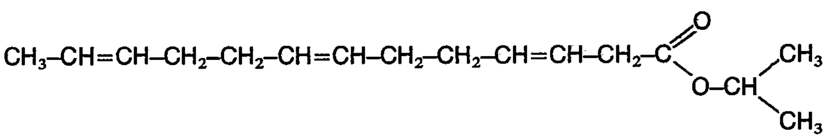

(c4)

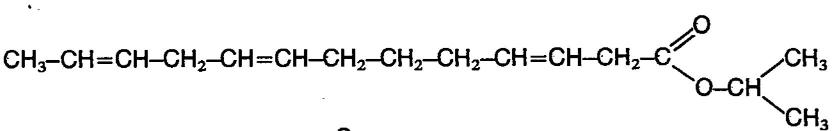

(c5)

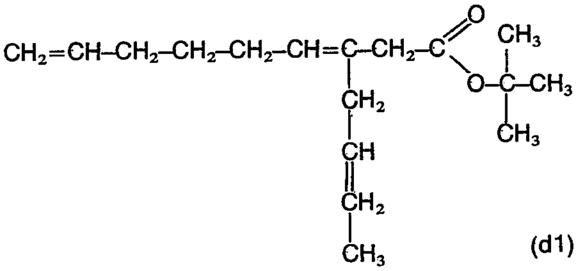

(d1)

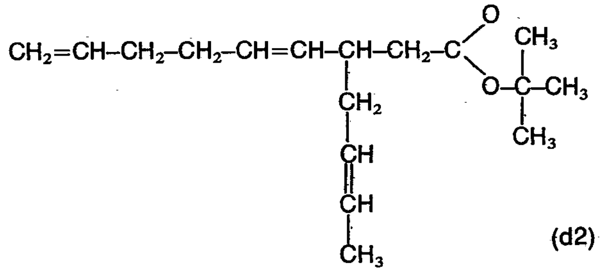

(d2)

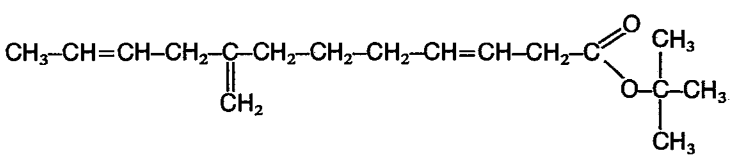

(d3)

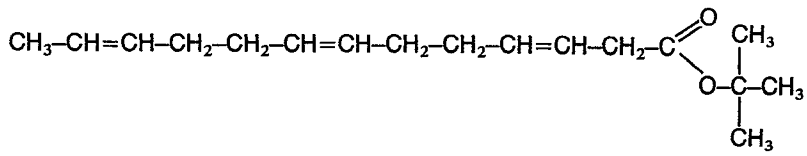

(d4)

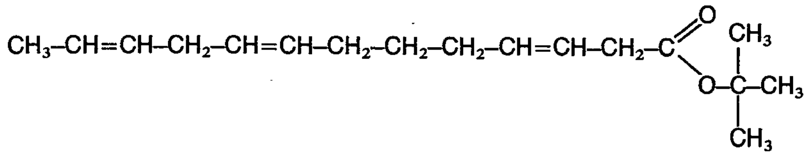

(d5)

Le procédé selon l'invention est mis en œuvre à une température généralement comprise entre environ 90 °C et environ 150 °C. On opère de préférence entre 100 environ et 130 °C environ.

La pression n'est pas un facteur critique. On opère habituellement sous pression atmosphérique bien qu'une pression inférieure ou supérieure à la pression atmosphérique ne soit pas exclue.

On opère généralement de la façon suivante: on utilise un réacteur de préférence en acier inoxydable dans lequel on introduit sous atmosphère inerte les réactifs préférentiellement, mais non obligatoirement, dans l'ordre suivant: composé du rhodium, composé trivalent du phosphor (si ce dernier est utilisé), alcool, composé IV et composé V. L'alcool qui est utilisé en faible quantité permet l'activation du système catalytique. On chauffe sous agitation. Lorsque la réaction est terminée, on élimine le composé V par dégazage. Les produits de la réaction sont ensuite isolés du mélange obtenu par distillation sous pression réduite.

Les composés suivant l'invention peuvent conduire soit par hydrogénation des doubles liaisons oléfiniques et saponification à des savons, soit par hydrogénation et hydrogénolyse à des alcools précurseurs de détergents biodégradables.

Les composés de départ de formule IV sont préparés selon les techniques de l'art antérieur. On peut, en particulier les préparer en faisant réagir du butadiène-1,3 dans un alcool sous presssion de CO et en présence d'un système catalytique composé d'un sel de palladium et de triphénylphosphine à des températures de l'ordre de 110 °C [voir par exemple Tetrahedron Vol. 28 page 3721 (1972)].

L'invention va maintenant être plus complètement décrite à l'aide des exemples suivants qui ne sauraient en aucune manière être considérés comme une limitation de l'invention.

Exemple 1

Réaction entre le nonadiène-3,8 oate d'éthyle et le butadiène-1,3 catalysée par Rh $Cl_3/C_2H_5OH$ sans triphényl-phosphine.

Dans un tube en verre de 40 $cm^3$, on introduit 0,043 g (0,12 milliatomegramme de Rh) de Rh $Cl_3$, x $H_2O$. On ferme le tube par un bouchon en caoutchouc (vaccine-cap) et on purge trois fois par un système vide-argon. On introduit successivement 0,3 g (6,5 millimoles) d'éthanol, 16,32 g (89,7 millimoles de nonadiène-3,8, oate d'éthyle et 5,22 g (96,6 millimoles) de butadiène-1,3. On scelle le tube et on l'introduit dans un autoclave de 500 ml qui est mis à chauffer à 120 °C sous agitation pendant 6 heures 30.

Le taux de transformation du butadiène est de 96,8% et celui du nonadiène-3,8 oate d'éthyle, de 76,5%.

Le couplage chromatographie en phase vapeur/spectrographie de masse ainsi que la RMN du proton et l'infra-rouge montre la présence des produits attendus $b_1$ $b_2$ $b_3$ $b_4$ $b_5$ dans le rapport molaire $b_1 + b_2 : b_3 : b_4 + b_5$ égal à 33/2,5/64,5. Plus précisément, on obtient:

- 2,72 g d'un mélange $b_1 + b_2$
- 0.2 g de $b_3$
- 5,33 g d'un mélange $b_4 + b_5$.

Exemple 2

Réaction entre le nonadiène-3,8 oate d'éthyle et le butadiène-1,3 catalysée par Rh $Cl_3/C_2H_5OH$ avec triphényl-phosphine (TPP).

$$\text{Rapport atomique } \frac{P^{+3}}{Rh} = 3$$

Dans un tube en verre de 40 $cm^3$, on introduit 0,043 g de Rh $Cl_3$, x $H_2O$ (0,12 milliatomegramme de Rh) et 0,097 g (0,36 millimole) de TPP. On ferme le tube par un bouchon en caoutchouc (vaccine-cap) et on purge trois fois par un système vide-argon. On introduit successivement 0,39 g (8,5 millimoles) d'éthanol, 15,8 g (87 millimoles) de nonadiène-3,8 oate d'éthyle et 5,1 g (94 millimoles) de butadiène. On scelle le tube et on l'introduit dans un autoclave de 500 ml qui est mis à chauffer à 120 °C sous agitation pendant 6 heures 30.

Le taux de tranformation du butadiène est de 79% et celui du nonadiène-3,8 oate d'éthyle, de 39,2%.

Par analyse chromatographique, on met en évidence les produits $b_1$ $b_2$ $b_3$ $b_4$ et $b_5$ dans le rapport molaire $b_1 + b_2 : b_3 : b_4 + b_5$ égal à 2/84/14. Plus précisément, on obtient:

- 0,13 g d'un mélange de $b_1 + b_2$
- 5,83 g de $b_3$
- 0,91 g d'un mélange de $b_4 + b_5$.

Cet exemple montre bien que l'addition de triphénylphosphine oriente la réaction vers la formation de $b_3$ tout en diminuant la quantité des lourds non distillables (3,5% dans l'exemple 2 au lieu de 30% dans l'exemple 1).

Exemples 3 à 5

On opère comme dans l'exemple 2 en utilisant les mêmes produits de départ et le même système catalytique mais on fait varier le rapport atomique $\frac{P^{+3}}{Rh}$. On obtient les résultats suivants:

| | Produits de départ | | $p^{3+}/Rh$ | TT % | TT % | Produits de la réaction | | |
|---|---|---|---|---|---|---|---|---|
| | Nonadiène-3,8 oate d'éthyle | Butadiène | | Butadiène | Nonadiène-3,8 oate d'éthyle | $b_1+b_2$ | $b_3$ | $b_4+b_5$ |
| Ex. 3 | 16,7 g | 5,22 g | 1 | 98,3 | 74,3 | 2,48 g | 1,42 g | 2,48 g |
| Ex. 4 | 15,63 g | 5,25 g | 2 | 87,4 | 61,2 | 0,34 g | 8,03 g | 2,82 g |
| Ex. 5 | 15,78 g | 5,58 g | 6 | 71,5 | 30 | 0 | 4,90 g | 1,15 g |

TT: Taux de transformation

Exemple 6

Réaction entre le nonandiène-3,8 oate d'isopropyle et le butadiène-1,3 catalysée par Rh $Cl_3/C_2H_5OH$ avec triphénylphosphine.

On opère comme dans l'exemple 2 mais en utilisant:

- 0,045 g de Rh $Cl_3$, x $H_2O$ (0,13 milliatomegramme de Rh)
- 0,111 g de triphénylphosphine (0,40 millimole)
- 0,18 g d'éthanol (3,9 millimoles)
- 16 g de nonadiène-3,8 oate d'isopropyle (82 millimoles)
- 5,3 g de butadiène (98 millimoles).

Le taux de transformation du butadiène est de 91% et celui du nonadiène-3,8 oate d'isopropyle est de 54%.

Par analyse chromatographique, on met en évidence les composés $c_1$ $c_2$ $c_3$ $c_4$ et $c_5$ dans le rapport molaire $c_1+c_2:c_3:c_4+c_5$ égal à 3/71/26. Plus précisément, on obtient:

- 0,32 g d'un mélange de $c_1+c_2$
- 7,2 g de $c_3$
- 2,65 g d'un mélange de $c_4+c_5$.

Exemples 7 et 8

On opère comme dans l'exemple 6 mais en faisant varier le rapport atomique $\frac{P^{+3}}{Rh}$. On obtient les résultats suivants:

| | Produits de départ | | $p^{3+}/Rh$ | TT % | TT % | Produits de la réaction | | |
|---|---|---|---|---|---|---|---|---|
| | Nonadiène-3,8 oate d'isoprop. | Butadiène | | Butadiène | Nonadiène-3,8 oate d'isoprop. | $c_1+c_2$ | $c_3$ | $c_4+c_5$ |
| Ex. 7 | 16,2 g | 4,9 | 0 | 98 | 66,5 | 2,24 g | 0,36 g | 5,89 g |
| Ex. 8 | 16,5 g | 5,5 | 4 | 70,6 | 44,5 | 0,1 g | 7,62 g | 1,46 g |

TT: Taux de transformation

Exemple 9

Réaction entre le nonadiène-3,8 oate de tertiobutyle et le butadiène-1,3 catalysée par Rh $Cl_3/C_2H_5OH$ avec triphénylphosphine.

On opère comme dans l'exemple 2 mais en utilisant:

- 0,045 g de Rh $Cl_3$ (0,13 milliatomegramme de Rh)
- 0,111 g de triphénylphosphine (0,40 millimole)
- 0,18 g d'éthanol (3,9 millimoles)
- 17,4 g de nonadiène-3,8 oate de tertiobutyle (83 millimoles)
- 4,98 g de butadiène (92 millimoles).

Le taux de transformation du butadiène est de 73% et celui du nonadiène-3,8 oate de tertiobutyle, de 48%.

Par analyse chromatographique, on met en évidence les composés $d_1$ $d_2$ $d_3$ $d_4$ et $d_5$ dans le rapport molaire $d_1+d_2:d_3:d_4+d_5$ égal à 2/76/22. Plus précisément, on obtient:

- 0,18 g d'un mélange de $d_1+d_2$
- 7,5 g de $d_3$
- 2,1 g d'un mélange de $d_4+d_5$.

Exemples 10 et 11

On opère comme dans l'exemple 9 mais en faisant varier le rapport atomique $\frac{P^{+3}}{Rh}$. On obtient les résultats suivants:

| | Produits de départ | | $p^{3+}$/Rh | TT % | TT % | Produits de la réaction | | |
|---|---|---|---|---|---|---|---|---|
| | Nonadiène-3,8 oate de tertio. | Butadiène | | Butadiène | Nonadiène-3,8 oate de tertio. | $d_1 + d_2$ | $d_3$ | $d_4 + d_5$ |
| Ex. 10 | 18 g | 5,4 g | 0 | 89,5 | 83 | 4,65 g | 0,52 g | 7,96 g |
| Ex. 11 | 17,4 g | 5,4 g | 4 | 79 | 48,5 | 0,15 g | 7,5 g | 2,2 g |

TT: Taux de transformation

**Revendications**
pour les états contractants: BE, CH, DE, FR, OB, IT, W, NL, SE.

1. Esters insaturés de formule A COO $R_1$ caractérisé en ce que $R_1$ représente un groupement alkyle ayant de 1 à 6 atomes de carbone et en ce que A est choisi parmi le groupe comprenant les restes hydrocarbonés de formule:

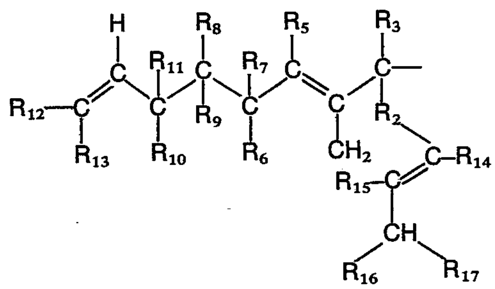

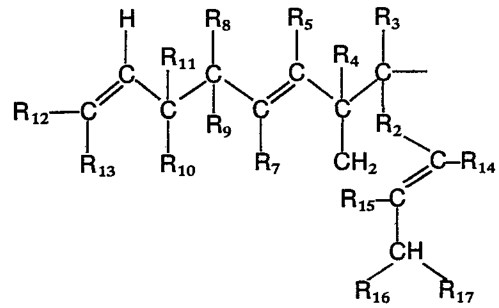

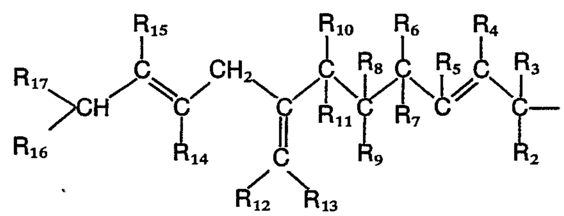

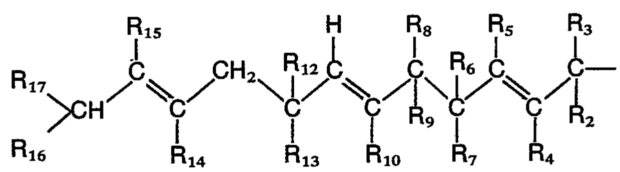

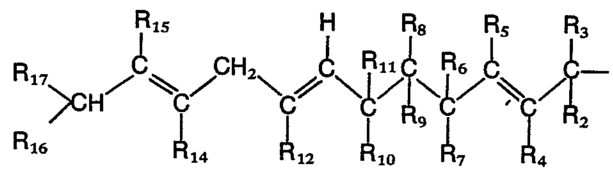

dans lesquels $R_2$ à $R_{17}$ identiques ou différents représentent un atome d'hydrogène ou un radical alkyle ayant de 1 à 3 atomes de carbone.

2. Ester selon la revendication 1 caractérisé en ce qu'il répond à la formule:

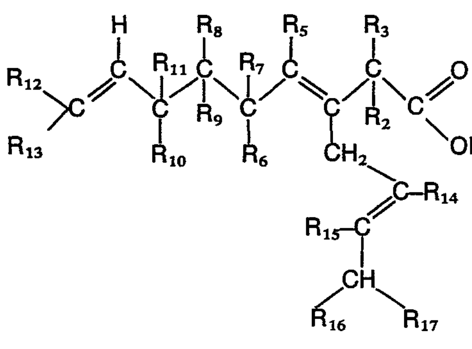

(Ia)

ou

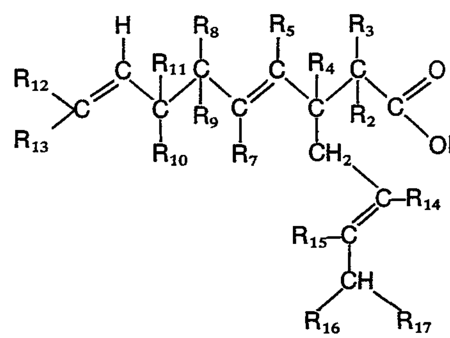

(Ib)

dans laquelle $R_1$ représente un radical alkyle ayant de 1 à 6 atomes de carbone et $R_2$ à $R_{17}$ identiques ou différents sont un atome d'hydrogène ou un radical alkyle ayant de 1 à 3 atomes de carbone.

3. Ester selon la revendication 1 caractérisé en ce qu'il répond à la formule:

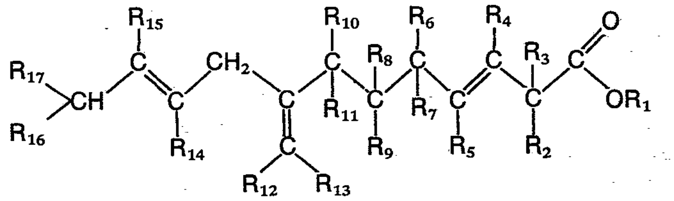

(II)

dans laquelle $R_1$ est un radical alkyle ayant de 1 à 6 atomes de carbone et $R_2$ à $R_{17}$ identiques ou différents sont un atome d'hydrogène ou un radical alkyle ayant de 1 à 3 atomes de carbone.

4. Ester selon la revendication 1 caractérisé en ce qu'il répond à la formule:

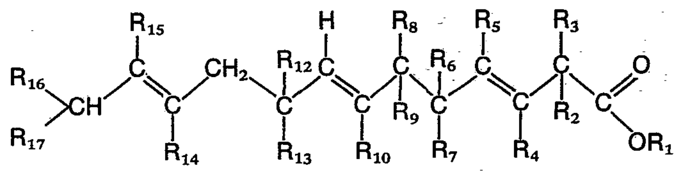

(IIIa)

ou

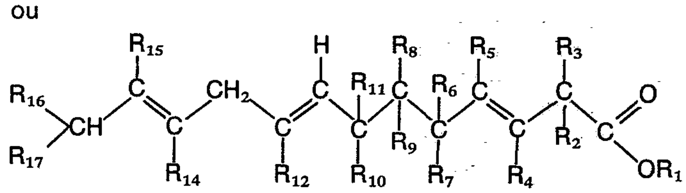

(IIIb)

dans lesquelles $R_1$ représente un radical alkyle ayant de 1 à 6 atomes de carbone et $R_2$ à $R_{17}$ identiques ou différents sont un atome d'hydrogène ou un radical alkyle ayant de 1 à 3 atomes de carbone.

5. Ester selon la revendication 2 caractérisé en ce que dans les formules Ia et Ib, $R_1$ représente un radical alkyle ayant de 1 à 4 atomes de carbone et $R_2$ à $R_{17}$ représentent chacun un atome d'hydrogène.

6. Ester selon la revendication 3 caractérisé en ce que dans la formule II, $R_1$ représente un radical alkyle ayant de 1 à 4 atomes de carbone et $R_2$ à $R_{17}$ représentent chacun un atome d'hydrogène.

7. Ester selon la revendication 4 caractérisé en ce que dans les formules (IIIa) et (IIIb), $R_1$ représente un radical alkyle ayant de 1 à 4 atomes de carbone et $R_2$ à $R_{17}$ représentant chacun un atome d'hydrogène.

8. Procédé de préparation d'esters selon la revendication 1 caractérisé en ce que l'on fait réagir un ester de formule:

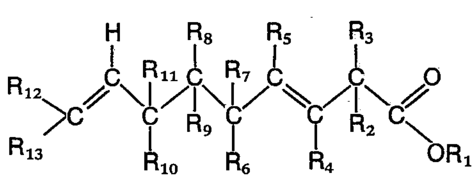

(IV)

dans laquelle $R_1$ représente un radical alkyle ayant de 1 à 6 atomes de carbone,

$R_2$ $R_3$ $R_5$ et $R_9$ identiques ou différents sont un atome d'hydrogène ou un radical alkyle ayant de 1 à 3 atomes de carbone,

$R_4$ $R_6$ et $R_7$ représentent un atome d'hydrogène ou un radical alkyle ayant de 1 à 3 atomes de carbone, au moins l'un d'entre eux étant un atome d'hydrogène,

$R_{10}$ $R_{11}$ $R_{12}$ et $R_{13}$ représentent un atome d'hydrogène ou un radical alkyle ayant de 1 à 3 atomes de carbone, au moins l'un d'entre eux étant un atome d'hydrogène, avec un butadiène-1,3 de formule:

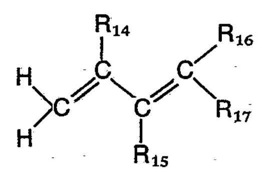

(V)

dans laquelle

$R_{14}$ $R_{15}$ $R_{16}$ ou $R_{17}$ identiques ou différents représentent chacu un atome d'hydrogène ou un radical alkyle, ayant de 1 à 3 atomes de carbone, en présence d'un composé du rhodium et d'un alcool.

9. Procédé selon la revendication 8 caractérisé en ce que l'on fait réagir un ester de formule IV dans laquelle $R_1$ représente un radical alkyle ayant de 1 à 4 atomes de carbone et $R_2$ à $R_{17}$ représentent chacun un atome d'hydrogène avec un composé de formule V dans laquelle $R_{14}$ à $R_{17}$ représentent un atome d'hydrogène.

10. Procédé selon l'une quelconque des revendications 8 et 9 caractérisé en ce que l'on met en œuvre simultanément au composé du rhodium et à l'alcool un composé trivalent du phosphore.

11. Procédé selon la revendication 10 caractérisé en ce que l'on met en œuvre le composé tri-

valent du phosphore en quantité telle que le rapport atomique

$$\frac{\text{phosphore trivalent}}{\text{rhodium}}$$

est supérieur ou égal à environ 1,5.

12. Procédé selon la revendication 11 caractérisé en ce que le rapport atomique

$$\frac{\text{phosphore trivalent}}{\text{rhodium}}$$

est égal à environ 3.

13. Procédé selon l'une quelconque des revendications 10 à 12 caractérisé en ce que le composé trivalent du phosphore est choisi parmi les aryl et alkylphosphines.

14. Procédé selon la revendication 13 caractérisé en ce que le composé trivalent du phosphore est la triphénylphosphine.

15. Procédé selon l'une quelconque des revendications 8 à 14 caractérisé en ce que l'on met en œuvre un composé du rhodium choisi parmi les sels d'acides inorganiques du rhodium.

16. Procédé selon la revendication 15 caractérisé en ce que le composé du rhodium est le chlorure de rhodium Rh $Cl_3$.

17. Procédé selon l'une quelconque des revendications 8 à 16 caractérisé en ce que l'on met en œuvre un alcool de formule R–OH dans laquelle R représente un radical alkyle ayant de 1 à 6 atomes de carbone.

18. Procédé selon la revendication 17 caractérisé en ce que l'alcool est l'alcool éthylique.

19. Procédé selon l'une quelconque des revendications 8 à 18 caractérisé en ce que le rapport molaire du composé de formule IV au composé du rhodium est compris entre environ 200 et environ 2000.

20. Procédé selon l'une quelconque des revendications 8 à 19 caractérisé en ce que le rapport molaire de l'alcool au composé du rhodium est compris entre environ 10 et environ 100.

21. Procédé selon l'une quelconque des revendications 8 à 20 caractérisé en ce que l'on opère à une température comprise entre environ 90 et environ 150 °C.

**Revendications**
pour l'état contractant: AT

1. Procédé de préparation d'esters insaturés de formule A COO $R_1$ dans laquelle $R_1$ représente un groupement alkyle ayant de 1 à 6 atomes de carbone et A est choisi parmi le groupe comprenant les restes hydrocarbonés de formule:

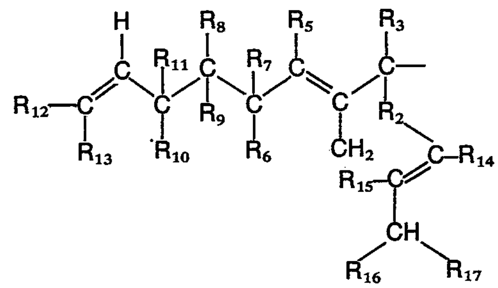

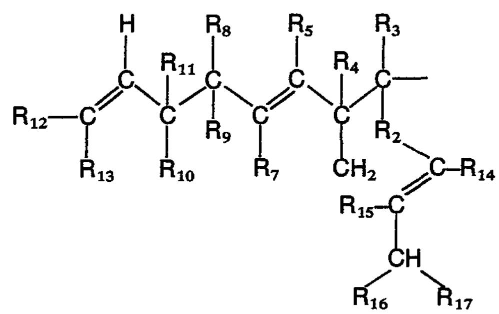

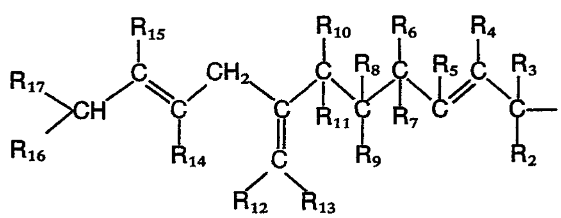

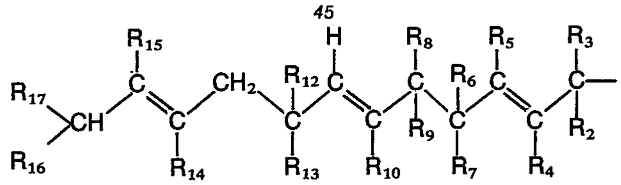

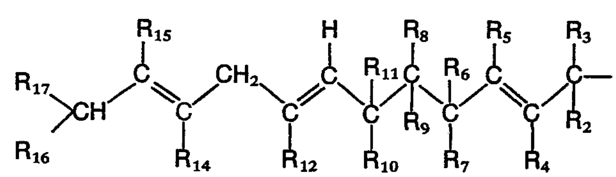

dans lesquels $R_2$ à $R_{17}$ identiques ou différents représentent un atome d'hydrogène ou un radical alkyle ayant de 1 à 3 atomes de carbone, caractérisé en ce que l'on fait réagir un ester de formule:

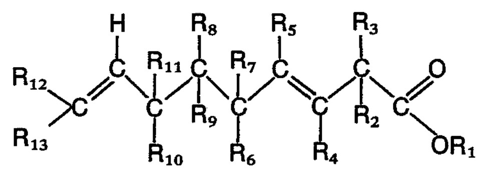

(IV)

dans laquelle les radicaux $R_1$ à $R_{13}$ ont la signification précédente, au moins un des radicaux $R_4$ $R_6$ et $R_7$ et au moins un des radicaux $R_{10}$ à $R_{13}$ étant un atome d'hydrogène, avec un butadiène-1,3 de formule:

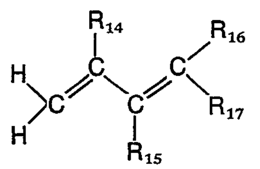

(V)

dans laquelle les radicaux $R_{14}$ à $R_{17}$ ont la signification précédente, en présence d'un composé du rhodium et d'un alcool.

2. Procédé selon la revendication 1 caractérisé en ce que l'on fait réagir un ester de formule IV dans laquelle $R_1$ représente un radical alkyle ayant de 1 à 4 atomes de carbone et $R_2$ à $R_{17}$ représentent chacun un atome d'hydrogène avec un composé de formule V dans laquelle $R_{14}$ à $R_{17}$ représentent un atome d'hydrogène.

3. Procédé selon l'une quelconque des revendications 1 à 2 caractérisé en ce que l'on en œuvre simultanément au composé du rhodium et à l'alcool un composé trivalent du phosphore.

4. Procédé selon la revendication 3 caractérisé en ce que l'on met en œuvre le composé trivalent du phosphore en quantité telle que le rapport atomique

$$\frac{\text{phosphore trivalent}}{\text{rhodium}}$$

est supérieur ou égal à environ 1,5.

5. Procédé selon la revendication 4 caractérisé en ce que le rapport atomique

$$\frac{\text{phosphore trivalent}}{\text{rhodium}}$$

est égal à environ 3.

6. Procédé selon l'une quelconque des revendications 3 à 5 caractérisé en ce que le composé trivalent du phosphore est choisi parmi les aryl et alkylphosphines.

7. Procédé selon la revendication 6 caractérisé en ce que le composé trivalent du phosphore est la triphénylphosphine.

8. Procédé selon l'une quelconque des revendications 1 à 7 caractérisé en ce que l'on met en œuvre un composé du rhodium choisi parmi les sels d'acides inorganiques du rhodium.

9. Procédé selon la revendication 8 caractérisé en ce que le composé du rhodium est le chlorure de rhodium $Rh\ Cl_3$.

10. Procédé selon l'une quelconque des revendications 1 à 9 caractérisé en ce que l'on met en œuvre un alcool de formule R–OH dans laquelle R représente un radical alkyle ayant de 1 à 6 atomes de carbone.

11. Procédé selon la revendication 10 caractérisé en ce que l'alcool est l'alcool éthylique.

12. Procédé selon l'une quelconque des revendications 1 à 11 caractérisé en ce que le rapport molaire du composé de formule IV au composé du rhodium est compris entre environ 200 et environ 2000.

13. Procédé selon l'une quelconque des revendications 1 à 12 caractérisé en ce que le rapport molaire de l'alcool au composé du rhodium est compris entre environ 10 et environ 100.

14. Procédé selon l'une quelconque des revendications précédentes caractérisé en ce que l'on opère à une température comprise entre environ 90 et environ 150°C.

**Patentansprüche**

für die Vertragsstaaten: BE, CH, DE, FR, GB, IT, LU, NL, SE

1. Ungesättigte Ester der Formel A COO $R_1$, dadurch gekennzeichnet, dass $R_1$ eine Alkylgruppe mit 1 bis 6 Kohlenstoffatomen bedeutet, und dass A ausgewählt ist aus der Gruppe umfassend die Kohlenwasserstoffreste der Formel

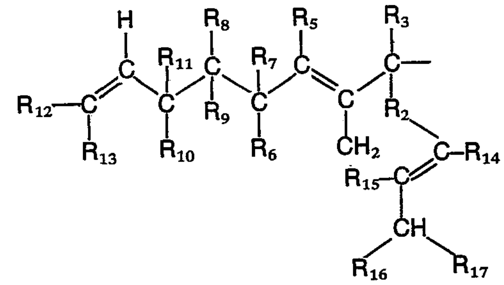

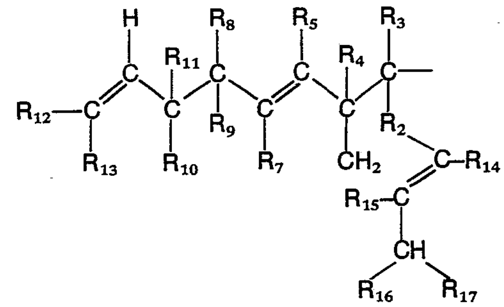

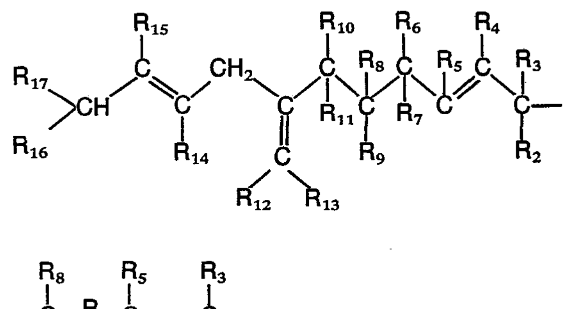

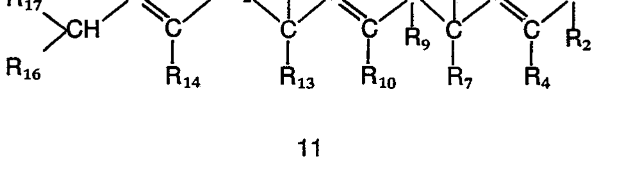

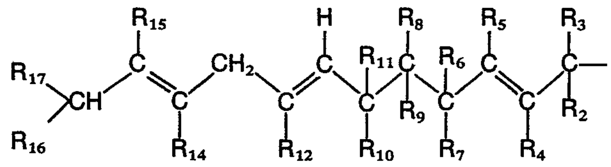

worin $R_2$ bis $R_{17}$, welche identisch oder voneinander verschieden sind, ein Wasserstoffatom oder einen Alkylrest mit 1 bis 3 Kohlenstoffatomen bedeuten.

2. Ester gemäss Anspruch 1, dadurch gekennzeichnet, dass er der Formel

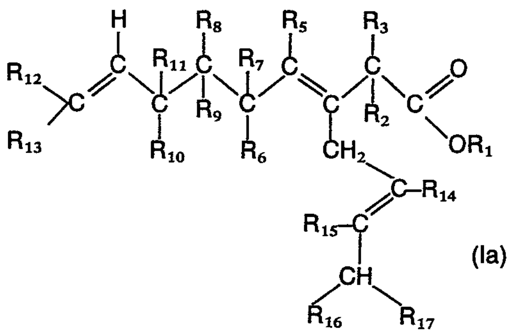

(Ia)

oder

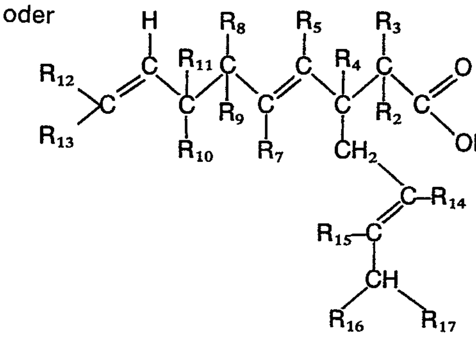

(Ib)

entspricht, worin $R_1$ einen Alkylrest mit 1 bis 6 Kohlenstoffatomen bedeutet und $R_2$ bis $R_{17}$, welche identisch oder voneinander verschieden sind, sind ein Wasserstoffatom oder ein Alkylrest mit 1 bis 3 Kohlenstoffatomen.

3. Ester gemäss Anspruch 1, dadurch gekennzeichnet, dass er der Formel

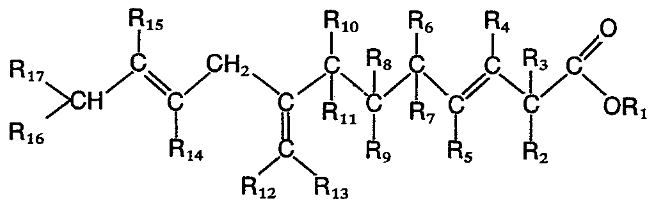

(II)

entspricht, worin $R_1$ ein Alkylrest mit 1 bis 6 Kohlenstoffatomen ist und $R_2$ bis $R_{17}$, welche identisch oder voneinander verschieden sind, ein Wasserstoffatom oder ein Alkylrest mit 1 bis 3 Kohlenstoffatomen sind.

4. Ester gemäss Anspruch 1, dadurch gekennzeichnet, dass er der Formel

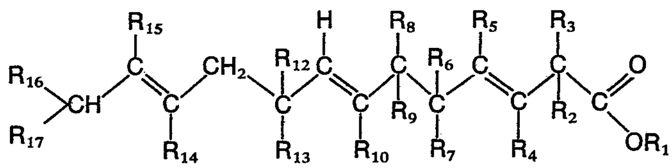

(IIIa)

oder

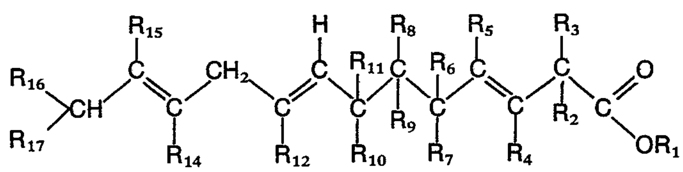

(IIIb)

entspricht, worin $R_1$ einen Alkylrest mit 1 bis 6 Kohlenstoffatomen bedeutet und $R_2$ bis $R_{17}$, welche identisch oder voneinander verschieden sind, ein Wasserstoffatom oder ein Alkylrest mit 1 bis 3 Kohlenstoffatomen sind.

5. Ester gemäss Anspruch 2, dadurch gekennzeichnet, dass in den Formeln Ia und Ib $R_1$ einen Alkylrest mit 1 bis 4 Kohlenstoffatomen bedeutet und $R_2$ bis $R_{17}$ jeweils ein Wasserstoffatom bedeuten.

6. Ester gemäss Anspruch 3, dadurch gekennzeichnet, dass in der Formel II $R_1$ einen Alkylrest mit 1 bis 4 Kohlenstoffatomen bedeutet und $R_2$ bis $R_{17}$ jeweils ein Wasserstoffatom bedeuten.

7. Ester gemäss Anspruch 4, dadurch gekennzeichnet, dass in den Formeln IIIa und IIIb $R_1$ einen Alkylrest mit 1 bis 4 Kohlenstoffatomen bedeutet und $R_2$ bis $R_{17}$ jeweils ein Wasserstoffatom bedeuten.

8. Verfahren zur Herstellung von Estern gemäss

Anspruch 1, dadurch gekennzeichnet, dass man einen Ester der Formel

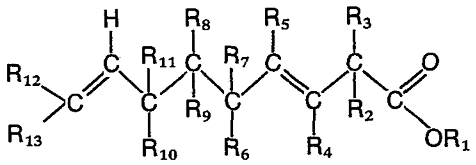

(IV)

worin $R_1$ einen Alkylrest mit 1 bis 6 Kohlenstoffatomen bedeutet,

$R_2$, $R_3$, $R_5$, $R_8$ und $R_9$, welche identisch oder verschieden sind, ein Wasserstoffatom oder ein Alkylrest mit 1 bis 3 Kohlenstoffatomen sind,

$R_4$, $R_6$ und $R_7$ ein Wasserstoffatom oder einen Alkylrest mit 1 bis 3 Kohlenstoffatomen darstellen, wobei wenigstens einer von diesen ein Wasserstoffatom ist,

$R_{10}$, $R_{11}$, $R_{12}$ und $R_{13}$ ein Wasserstoffatom oder einen Alkylrest mit 1 bis 3 Kohlenstoffatomen bedeuten, wobei wenigstens einer von ihnen ein Wasserstoffatom ist, mit einem 1,3-Butadien der Formel

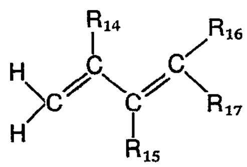

(V)

worin $R_{14}$, $R_{15}$, $R_{16}$ oder $R_{17}$, welche identisch oder voneinander verschieden sind, jeweils ein Wasserstoffatom oder einen Alkylrest mit 1 bis 3 Kohlenstoffatomen bedeuten, in Gegenwart einer Verbindung des Rhodiums und eines Alkohols zur Reaktion bringt.

9. Verfahren gemäss Anspruch 8, dadurch gekennzeichnet, dass man einen Ester der Formel IV, worin $R_1$ einen Alkylrest mit 1 bis 4 Kohlenstoffatomen bedeutet und $R_2$ bis $R_{17}$ jeweils ein Wasserstoffatom bedeuten, mit einer Verbindung der Formel V, worin $R_{14}$ bis $R_{17}$ ein Wasserstoffatom bedeuten, zur Reaktion bringt.

10. Verfahren gemäss einem der Ansprüche 8 und 9, dadurch gekennzeichnet, dass man gleichzeitig mit der Verbindung des Rhodiums und dem Alkohol eine dreiwertige Verbindung des Phosphors einsetzt.

11. Verfahren gemäss Anspruch 10, dadurch gekennzeichnet, dass man die dreiwertige Verbindung des Phosphors in einer solchen Menge einsetzt, dass das Atomverhältnis

$$\frac{\text{dreiwertiger Phosphor}}{\text{Rhodium}}$$

grösser oder gleich etwa 1,5 ist.

12. Verfahren gemäss Anspruch 11, dadurch gekennzeichnet, dass das Atomverhältnis

$$\frac{\text{dreiwertiger Phosphor}}{\text{Rhodium}}$$

gleich etwa 3 ist.

13. Verfahren gemäss einem der Ansprüche 10 bis 12, dadurch gekennzeichnet, dass die dreiwertige Verbindung des Phosphors ausgewählt ist unter den Aryl- und Alkylphosphinen.

14. Verfahren gemäss Anspruch 13, dadurch gekennzeichnet, dass die dreiwertige Verbindung des Phosphors das Triphenylphosphin ist.

15. Verfahren gemäss einem der Ansprüche 8 bis 14, dadurch gekennzeichnet, dass man eine Verbindung des Rhodiums einsetzt, die ausgewählt ist unter den Salzen anorganischer Säuren des Rhodiums.

16. Verfahren gemäss Anspruch 15, dadurch gekennzeichnet, dass die Verbindung des Rhodiums das Rhodiumchlorid Rh $Cl_3$ ist.

17. Verfahren gemäss einem der Ansprüche 8 bis 16, dadurch gekennzeichnet, dass man einen Alkohol der Formel R–OH einsetzt, worin R einen Alkylrest mit 1 bis 6 Kohlenstoffatomen bedeutet.

18. Verfahren gemäss Anspruch 17, dadurch gekennzeichnet, dass der Alkohol Äthylalkohol ist.

19. Verfahren gemäss einem der Ansprüche 8 bis 18, dadurch gekennzeichnet, dass das Molverhältnis der Verbindung der Formel IV zur Verbindung des Rhodiums zwischen etwa 200 und etwa 2000 liegt.

20. Verfahren gemäss einem der Ansprüche 8 bis 19, dadurch gekennzeichnet, dass das Molverhältnis des Alkohols zur Verbindung des Rhodiums zwischen etwa 10 und etwa 100 liegt.

21. Verfahren gemäss einem der Ansprüche 8 bis 20, dadurch gekennzeichnet, dass man bei einer Temperatur zwischen etwa 90 und 150°C arbeitet.

**Patentansprüche**
für den Vertragsstaat: AT

1. Verfahren zur Herstellung ungesättigter Ester der Formel A COO $R_1$, worin $R_1$ eine Alkylgruppe mit 1 bis 6 Kohlenstoffatomen bedeutet und A ausgewählt ist unter den Gruppen, umfassend die Kohlenwasserstoffreste der Formel

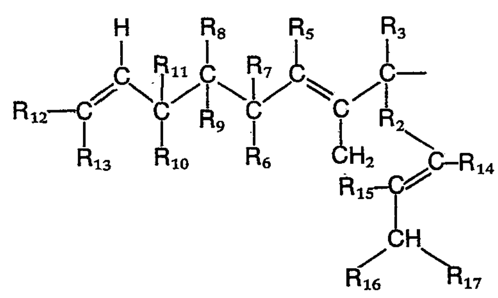

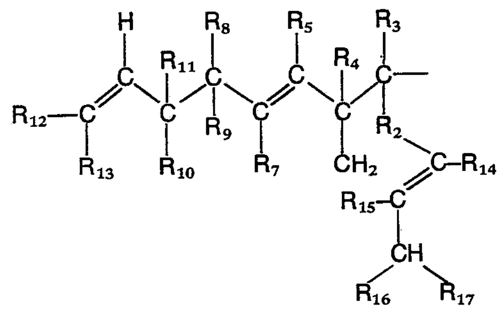

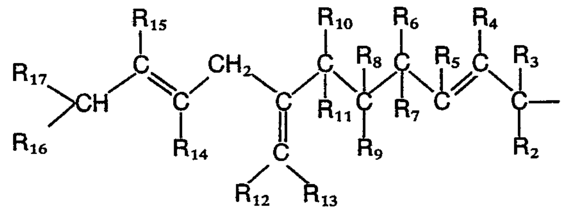

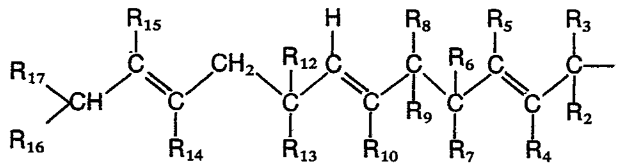

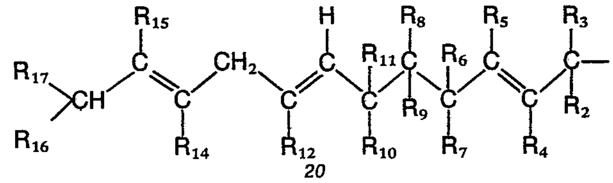

worin $R_2$ bis $R_{17}$, welche identisch oder verschieden sind, ein Wasserstoffatom oder einen Alkylrest mit 1 bis 3 Kohlenstoffatomen bedeuten, dadurch gekennzeichnet, dass man einen Ester der Formel IV

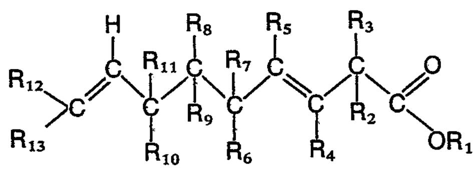

(IV)

worin die Reste $R_1$ bis $R_{17}$ die vorstehende Bedeutung haben, wobei mindestens einer der Reste $R_4$, $R_6$ und $R_7$ und mindestens einer der Reste $R_{10}$ bis $R_{13}$ ein Wasserstoffatom ist, mit einem 1,3-Butadien der Formel

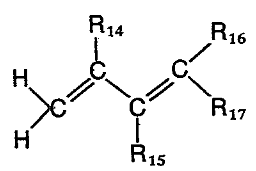

(V)

worin die Reste $R_{14}$ bis $R_{17}$ die vorstehende Bedeutung besitzen, in Gegenwart einer Verbindung des Rhodiums und eines Alkohols zur Reaktion bringt.

2. Verfahren gemäss Anspruch 1, dadurch gekennzeichnet, dass man einen Ester der Formel IV, worin $R_1$ einen Alkylrest mit 1 bis 4 Kohlenstoffatomen bedeutet und $R_2$ bis $R_{17}$ jeweils ein Wasserstoffatom bedeuten, mit einer Verbindung der Formel V, worin $R_{14}$ bis $R_{17}$ ein Wasserstoffatom bedeuten, zur Reaktion bringt.

3. Verfahren gemäss einem der Ansprüche 1 und 2, dadurch gekennzeichnet, dass man gleichzeitig mit der Verbindung des Rhodiums und dem Alkohol eine dreiwertige Verbindung des Phosphors einsetzt.

4. Verfahren gemäss Anspruch 3, dadurch gekennzeichnet, dass man die dreiwertige Verbindung des Phosphors in einer solchen Menge einsetzt, dass das Atomverhältnis

$$\frac{\text{dreiwertiger Phosphor}}{\text{Rhodium}}$$

grösser oder gleich etwa 1,5 ist.

5. Verfahren gemäss Anspruch 4, dadurch gekennzeichnet, dass das Atomverhältnis

$$\frac{\text{dreiwertiger Phosphor}}{\text{Rhodium}}$$

gleich etwa 3 ist.

6. Verfahren gemäss einem der Ansprüche 3 bis 5, dadurch gekennzeichnet, dass die dreiwertige Verbindung des Phosphors ausgewählt ist unter den Aryl- und Alkylphosphinen.

7. Verfahren gemäss Anspruch 6, dadurch gekennzeichnet, dass die dreiwertige Verbindung des Phosphors das Triphenylphosphin ist.

8. Verfahren gemäss einem der Ansprüche 1 bis 7, dadurch gekennzeichnet, dass man eine Verbindung des Rhodiums einsetzt, ausgewählt unter den Salzen anorganischer Säuren des Rhodiums.

9. Verfahren gemäss Anspruch 8, dadurch gekennzeichnet, dass die Verbindung des Rhodiums das Rhodiumchlorid Rh $Cl_3$ ist.

10. Verfahren gemäss einem der Ansprüche 1 bis 9, dadurch gekennzeichnet, dass man einen Alkohol der Formel R–OH einsetzt, worin R einen Alkylrest mit 1 bis 6 Kohlenstoffatomen bedeutet.

11. Verfahren gemäss Anspruch 10, dadurch gekennzeichnet, dass der Alkohol der Äthylalkohol ist.

12. Verfahren gemäss einem der Ansprüche 1 bis 11, dadurch gekennzeichnet, dass das Molverhältnis der Verbindung der Formel IV zur Verbindung des Rhodiums zwischen etwa 200 und etwa 2000 liegt.

13. Verfahren gemäss einem der Ansprüche 1 bis 12, dadurch gekennzeichnet, dass das Molverhältnis des Alkohols zur Verbindung des Rhodiums zwischen etwa 10 und etwa 100 liegt.

14. Verfahren gemäss einem der vorhergehenden Ansprüche, dadurch gekennzeichnet, dass man bei einer Temperatur zwischen etwa 90 und 150 °C arbeitet.

**Claims**

for the Contracting States: BE, CH, DE, FR, GB, IT, LU, NL, SE

1. Unsaturated esters of the formula A COO $R_1$, characterized in that $R_1$ represents an alkyl group having from 1 to 6 carbon atoms and in that A is chosen from amongst the group comprising the hydrocarbon radicals of the formula:

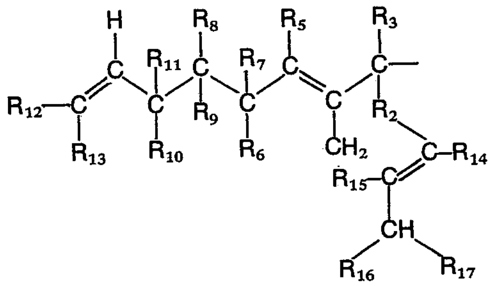

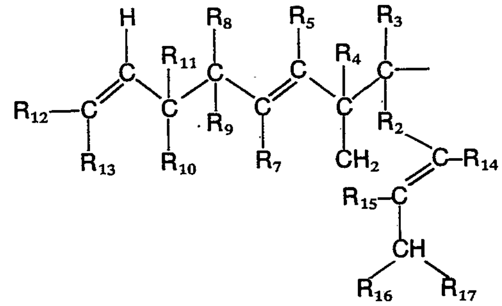

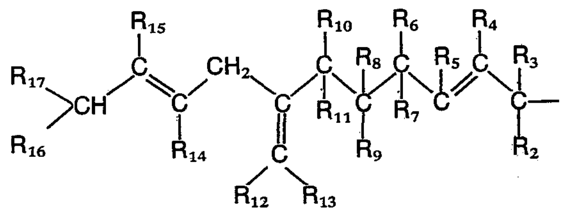

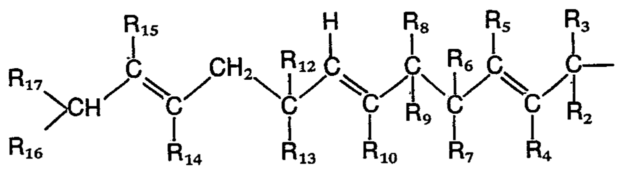

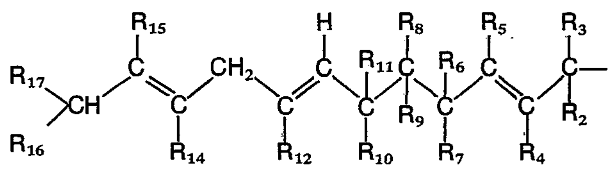

in which radicals $R_2$ to $R_{17}$, which are identical or different, represent a hydrogen atom or an alkyl radical having from 1 to 3 carbon atoms.

2. Esters according to claim 1, characterized in that it corresponds to the formula:

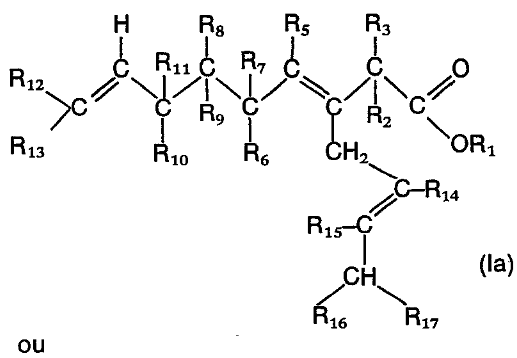

(Ia)

ou

or

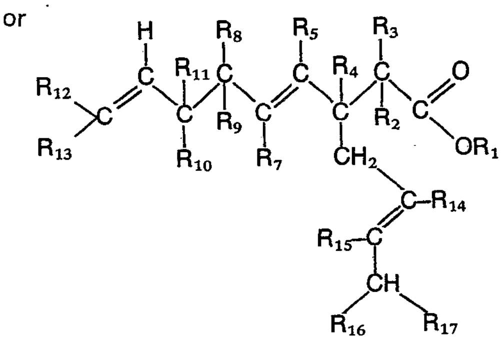

(Ib)

in which $R_1$ represents an alkyl radical having from 1 to 6 carbon atoms and $R_2$ to $R_{17}$, which are identical or different, are a hydrogen atom or an alkyl radical having from 1 to 3 carbon atoms.

3. Ester according to claim 1, characterized in that it corresponds to the formula:

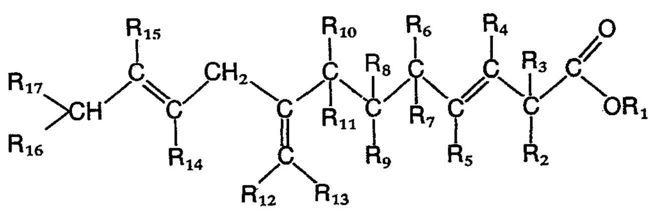

(II)

in which $R_1$ is an alkyl radical having from 1 to 6 carbon atoms and $R_2$ to $R_{17}$, which are identical or different, are a hydrogen atom or an alkyl radical having from 1 to 6 carbon atoms.

4. Ester according to claim 1, characterized in that it corresponds to the formula:

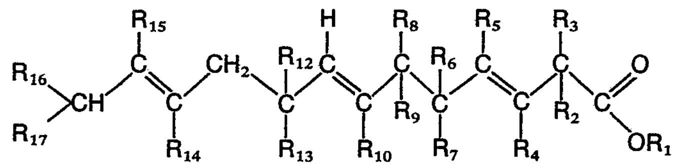

(IIIa)

or

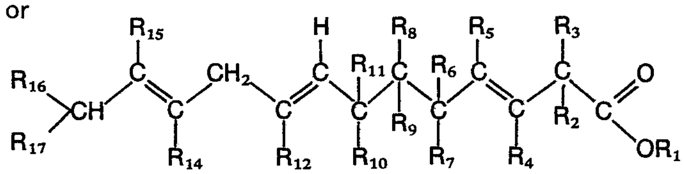

(IIIb)

in which formulae $R_1$ represents an alkyl radical having from 1 to 6 carbon atoms and $R_2$ to $R_{17}$, which are identical or different, are a hydrogen atom or an alkyl radical having from 1 to 3 carbon atoms.

5. Ester according to claim 2, characterized in that, in the formulae Ia and Ib, $R_1$ represents an alkyl radical having from 1 to 4 carbon atoms and $R_2$ to $R_{17}$ each represent a hydrogen atom.

6. Ester according to claim 3, characterized in that, in the formula II, $R_1$ represents an alkyl radical having from 1 to 4 carbon atoms and $R_2$ to $R_{17}$ each represent a hydrogen atom.

7. Ester according to claim 4, characterized in that, in the formulae (IIIa) and (IIIb), $R_1$ represents an alkyl radical having from 1 to 4 carbon atoms and $R_2$ to $R_{17}$ each represent a hydrogen atom.

8. Process for the preparation of esters according to claim 1, characterized in that an ester of the formula:

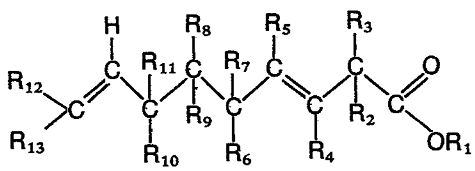

(IV)

in which $R_1$ represents an alkyl radical having from 1 to 6 carbon atoms, $R_2$, $R_3$, $R_5$, $R_8$ and $R_9$, which are identical of different, are a hydrogen atom or an alkyl radical having from 1 to 3 carbon atoms, $R_4$, $R_6$ and $R_7$ represents a hydrogen atom or an alkyl radical having from 1 to 3 carbon atoms, at least one of them being a hydrogen atom, and $R_{10}$, $R_{11}$, $R_{12}$ and $R_{13}$ represent a hydrogen atom or an alkyl radical having from 1 to 3 carbon atoms, at least one of them being a hydrogen atom, is reacted with a buta-1,3-diene of the formula:

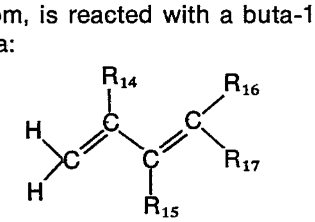

(V)

in which $R_{14}$, $R_{15}$, $R_{16}$ or $R_{17}$, which are identical or different, each represent a hydrogen atom or an alkyl radical having from 1 to 3 carbon atoms, in the presence of a rhodium compound and an alcohol.

9. Process according to claim 8, characterized in that an ester of the formula IV in which $R_1$ represent an alkyl radical having from 1 to 4 carbon atoms and $R_2$ to $R_{17}$ each represent a hydrogen atom is reacted with a compound of the formula V in which $R_{14}$ to $R_{17}$ represent a hydrogen atom.

10. Process according to either one of claims 8 and 9, characterized in that a trivalent phosphorus compound is used simultaneaously with the rhodium compound and the alcohol.

11. Process according to claim 10, characterized in that the trivalent phosphorus compound is used in an amount such that the atomic ratio

$$\frac{\text{trivalent phosphorus}}{\text{rhodium}}$$

is greater than or equal to about 1,5.

12. Process according to claim 11, characterized in that the atomic ratio

$$\frac{\text{trivalent phosphorus}}{\text{rhodium}}$$

is equal to about 3.

13. Process according to any one of claims 10 to 12, characterized in that the trivalent phosphorus compound is chosen from amongst aryl- and alkyl-phosphines.

14. Process according to claim 13, characterized in that the trivalent phosphorus compound is triphenylphosphine.

15. Process according to any one of claims 8 to 14, characterized in that a rhodium compound chosen from amongst inorganic acid salts of rhodium is used.

16. Process according to claim 15, characterized in that the rhodium compound is rhodium chloride, $Rh\ Cl_3$.

17. Process according to any one of claims 8 to 16, characterized in that an alcohol of the formula R–OH in which R represents an alkyl radical having from 1 to 6 carbon atoms is used.

18. Process according to claim 17, characterized in that the alcohol is ethyl alcohol.

19. Process according to any one of claims 8 to 18, characterized in that the molar ratio of the compound of the formula IV to the rhodium compound is between about 200 and about 2000.

20. Process according to any one of claims 8 to 19, characterized in that the molar ratio of the alcohol to the rhodium compound is between about 10 and about 100.

21. Process according to any claims 8 to 20, characterized in that the reaction is carried out at a temperature between about 90 an about 150 °C.

**Claims**
for the Contracting State: AT

1. Process for the preparation of unsaturated esters of the formula A COO $R_1$, in which $R_1$ represents an alkyl group having from 1 to 6 carbon atoms and A is chosen from amongst the group comprising the hydrocarbon radicals of the formula:

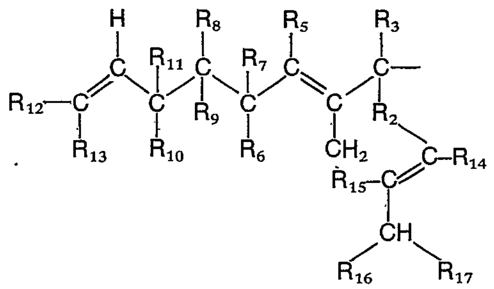

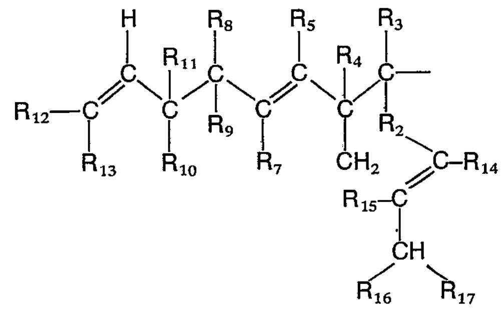

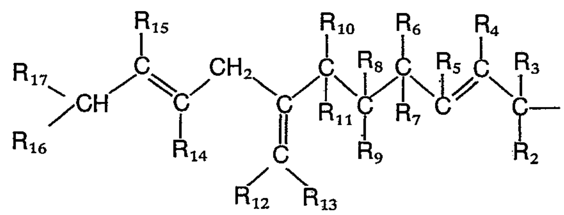

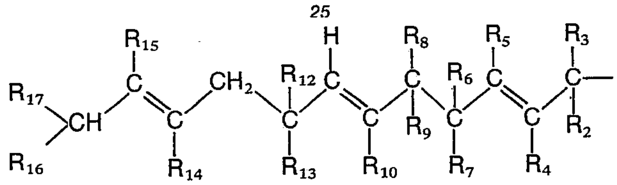

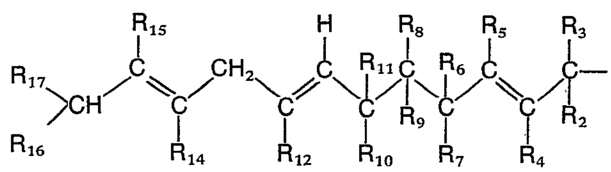

in which radicals $R_2$ to $R_{17}$, which are identical or different, represent a hydrogen atom or an alkyl radical having from 1 to 3 carbon atoms, characterized in that an ester of the formula:

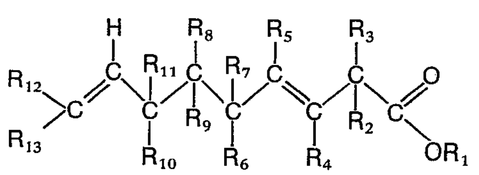
(IV)

in which the radicals $R_1$ to $R_{13}$ have the above meaning, at least one of the radicals $R_4$, $R_6$ and $R_7$ and at least one of the radicals $R_{10}$ to $R_{13}$ being a hydrogen atom, is reacted with a buta-1,3-diene of the formula:

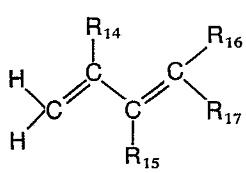
(V)

in which the radicals $R_{14}$ to $R_{17}$ have the above meaning, in the presence of a rhodium compound and an alcohol.

2. Process according to claim 1, characterized in that an ester of the formula IV in which $R_1$ represents an alkyl radical having from 1 to 4 carbon atoms and $R_2$ to $R_{17}$ each represent a hydrogen atom is reacted with a compound of the formula V in which $R_{14}$ to $R_{17}$ represent a hydrogen atom.

3. Process according to either one of claims 1 and 2, characterized in that a trivalent phosphorus compound is used simultaneously with the rhodium compound and the alcohol.

4. Process according to claim 3, characterized in that the trivalent phosphorus compound is used in an amount such that the atomic ratio

$$\frac{\text{trivalent phosphorus}}{\text{rhodium}}$$

is greater than or equal to about 1,5.

5. Process according to claim 4, characterized in that the atomic ratio

$$\frac{\text{trivalent phosphorus}}{\text{rhodium}}$$

is equal to about 3.

6. Process according to any one of claims 3 to 5, characterized in that the trivalent phosphorus compound is chosen from amongst aryl- and alkyl-phosphines.

7. Process according to claim 6, characterized in that the trivalent phosphorus compound is triphenylphosphine.

8. Process according to any one of claims 1 to 7, characterized in that a rhodium compound chosen from amongst inorganic acid salts of rhodium is used.

9. Process according to claim 8, characterized in that the rhodium compound is rhodium chloride, $Rh\ Cl_3$.

10. Process according to any one of claims 1 to 9, characterized in that an alcohol of the formula R–OH in which R represents an alkyl radical having from 1 to 6 carbon atoms is used.

11. Process according to claim 10, characterized in that the alcohol is ethyl alcohol.

12. Process according to any one of claims 1 to 11, characterized in that the molar ratio of the compound of the formula IV to the rhodium compound is between about 200 and about 2000.

13. Process according to any one of claims 1 to 12, charaterized in that the molar ratio of the alcohol to the rhodium compound is between about 10 and about 100.

14. Process according to any one of the preceding claims, characterized in that the reaction is carried out at a temperature between about 90 and about 150°C.